Europäisches Patentamt

**European Patent Office**

Office européen des brevets

(11) Publication number: **0 355 200**
**A1**

## (12) EUROPEAN PATENT APPLICATION

(21) Application number: 88117851.1

(51) Int. Cl.⁴: **A61B 17/22**

(22) Date of filing: 26.10.88

(30) Priority: 12.08.88 US 231779

(43) Date of publication of application:
28.02.90 Bulletin 90/09

(84) Designated Contracting States:
CH DE FR GB IT LI NL

(71) Applicant: ADVANCED CARDIOVASCULAR
SYSTEMS, INC.
1395 Charleston Road
Mountain View, CA 94039-7101(US)

(72) Inventor: Samson, Gene L.
32916 Great Salt Lake Street
Fremont, California 94587(US)
Inventor: Aita, Michael
1043 Payette Avenue
Sunnyvale, California 94087(US)

(74) Representative: Baillie, Iain Cameron et al
c/o Ladas & Parry Isartorplatz 5
D-8000 München 2(DE)

(54) Balloon dilatation catheter with laser cutting capability.

(57) An intravenous catheter (11) with laser cutting
capabilities includes a flexible elongate member (12)
containing at least one lumen (16). A fiber optic
assembly (21) extending through the lumen termi-
nates in a lens (31) at its distal end. The lens is
substantially larger in diameter than the diameter of
the fiber optic member and the transition area be-
tween the lens and fiber optic member is reinforced
with a collar (34) that is adhesively bonded to the
lens and fiber. An inflatable balloon (41) may be
carried by the distal end of the tubular member and
may be used for balloon angioplasty after a laser
cutting proceedure to remove a portion of stenosis in
the patients vasculature. The balloon is inflated and
deflated by a flow passage (43) located in the tubu-
lar member. Means to enhance rigidity and pushabil-
ity (22) are also provided in the tubular member.

FIG. 2

# BALLOON DILATATION CATHETER WITH LASER CUTTING CAPABILITY

## BACKGROUND OF THE INVENTION

### Field of the Invention

This invention relates to intravenous catheters and more particularly to intravenous catheters having laser cutting capabilities.

### Description of Related Art

Intravenous catheter systems have been developed for a variety of medical procedures. One particular area of interest in modern medicine has been the development of catheters adaptable for various angioplasty tasks associated with the reduction of stenosis in the coronary arteries. Catheter systems proposed for these purposes have included balloon angioplasty catheters in which a circumferential balloon on the catheter is inflated after insertion into the stenosis, thereby increasing the area available for blood flow, and laser angioplasty apparatus adaptable to destroy plaque by the use of a high energy light beam.

Balloon dilatation techniques have proven to be quite effective and relatively safe for these purposes and have assumed a respected place among the tools available to treat cardiovascular disease. However, balloon angioplasty techniques are generally limited in that they do not destroy the plaque, but press it out of the center of the occluded channel and may be dangerous for certain patients that have weakened vasculature or calcified plaque. Also, if a channel is occluded to the extent that the deflated balloon and catheter may not be inserted through the stenosis, it may be impossible to perform a balloon dilatation procedure.

In an attempt to overcome these limitations and provide a more discriminating procedure for angioplasty, laser angioplasty systems have been proposed. Such systems incorporate an optical fiber assembly to conduct laser energy from a remote location to the area of stenosis through an otherwise conventional catheter. In a number of cases, a balloon has been added to prevent the flow of blood during the procedure. While such systems were initially greeted with enthusiasm and held great promise, they were later seen to have important limitations. Among these limitations were the inability to direct the energy to an exact location to be dissolved, with the result that damage could be sustained by the artery wall due to misdirected radiation. Also, it was found that the exact mechanism of the reduction of plaque (whether light energy, heat or a combination) was not well known or easily predicted. Also, it was found that the means used to attach the lens to the end of the optical fiber had important ramifications regarding the amount of energy that could be propagated without destroying or dislodging the lens. Additionally, the means of attaching the lens to the fiber had an important effect on the probability that the lens would become dislodged, damaged or misaligned from handling on its passage through the artery.

There remains, therefore, a need for an intravenous angioplasty system that allows the accurate direction of laser energy to a stenosis, takes advantage of the most important of the mechanisms available from the laser energy to reduce an obstruction, minimizes the chance of damage to the healthy parts of the artery, and reduces the chance of damage to the patient from dislodging or disintegrating of the lens end of the optical fiber. It would also be beneficial if such a system could easily be combined with catheter configurations that could be used for allied procedures.

## SUMMARY OF THE INVENTION

The present invention provides a laser angioplasty catheter which substantially improves the directability and effectiveness of the beam of light propagating from the end of an optical fiber incorporated to conduct the light from a remote laser to an area of stenosis in a patient's vasculature. Furthermore, the construction of a laser angioplasty catheter according to the invention substantially improves the resistance of the lens end of the fiber to degradation or fracture due to mishandling or transfer of heat and improves the pushability of the catheter in use.

The invention provides a lens end in combination with an optical fiber used to conduct light from a remote laser to an area of stenosis. The lens end is substantially larger than the fiber diameter and provides a focused beam in a coaxial direction with the center of the lens. In practice, it has been found that a transparent, essentially spherical lens, or bead, that is fused with the end of the fiber and which is substantially larger, e.g., two to ten times the diameter of the fiber, provides unexpected and highly beneficial advantages compared to flat lenses that are extensions to the fiber or curved lenses that are nearly the same diameter as the fiber. In order to provide a more reliable and durable structure for the fiber-lens assembly, and to improve the pushability of the assembly and due to the relatively large diameter of the ball compared to the fiber, a reinforcing collar is provided that reinforces the area where the lens is fused to the fiber and extends around a portion of the lens. The collar also provides a means to assist in the control of thermal gradients across the interface between the lens and the fiber and may be configured to assist in the focusing of light energy in the desired pattern.

The construction of the lens-fiber system of the present invention provides numerous benefits compared to lens-fiber systems in which the lens is nearly the same diameter as the fiber. One such advantage is that the construction of the present invention assists in guiding the end of the optical fiber through the tortuous coronary arterial branches by presenting a larger area to bear against the wall of the vessel. This benefit is enhanced by other features of the invention that increase the pushability of the fiber and by reinforcing the area near the interface between the fiber and the lens.

In use, the invention allows the opening of a channel in even a heavily occluded artery by use of the laser light impinging on the stenosis. Thereafter, the balloon dilatation feature of the invention may be employed to perform a balloon angioplasty procedure after a channel large enough to admit the body of the catheter has been made by the laser beam. Thus, the use of the present invention provides a larger recanalization path than previous systems, with a lower probability of perforating the vessel wall. It can also be readily seen that the combination of features presented by the present invention substantially improves the effectiveness of a laser angioplasty procedure, especially in combination with a balloon angioplasty feature.

It has been found that the lens may be advantageously formed with a spherical surface and that such a shape focuses the light energy coaxially with the lens and fiber a distance approximately one to five lens diameters in front of the lens with the focal length and the energy distribution a function of the relationship between the lens diameter and fiber diameter, with the energy dissipating in a conical pattern therefrom. In practice, it has been found that the present invention is effective if placed in contact with the stenosis, since the short focal length of the lens-fiber system allows concentration of the energy directly in front of the lens. Another advantage to the configuration of the present invention is that the lens may be advanced through an area of stenosis with lessened danger of damaging the wall of the vessel due to wide angle propagation of the light energy.

By using the lens in contact with the stenosis, essentially all of the laser energy is directed into the central region of the stenosis and the catheter may be advanced as the plaque is dissolved. Also, the larger lens size results in a larger opening in the stenosis through which the catheter may be advanced.

The invention incorporates lens ends that are fused with the distal end of the optical fiber. It has been found that such lens ends may be formed on the distal end of the fiber by either fusing a transparent glass bead to the end of the fiber in the presence of an electric arc or by forming a spherical bead on the end of the fiber by melting the end of the fiber in a low residual flame. To improve the performance of the system, the indices of refraction of the optical fiber core and any lens attached may be matched. In practice, it has been found that this means of attaching the lens to an optical fiber, in combination with a reinforcing collar which abuts the bead and extends over the fiber and is adhesively attached to both the fiber and the bead, provides a robust connection that lowers the optical losses between the fiber and the lens and substantially reduces the possibility of fracture of the connection between the lens and the fiber compared to other methods. These effects are enhanced in the present invention, which utilizes matched indices of refraction in the lens and the optical fiber to further reduce losses and control light propagation. Thus, the configuration and method of attachment of the lens of the present invention provides important benefits in the use of fiber optics for in vivo angioplasty.

From the above it can be seen that the present invention provides many benefits in the field of angioplasty and that such benefits are enhanced when used in combination with balloon angioplasty catheter systems. Other features and advantages of the present invention will become apparent from the following detailed description, taken in conjunction with the accompanying drawings, which illustrate, by way of example, the principles of the invention.

BRIEF DESCRIPTION OF THE DRAWINGS

FIGURE 1 is a side elevational view of a balloon dilatation catheter with laser cutting capabilities incorporated in the present invention;

FIGURE 2 is an enlarged cross-sectional view of distal extremity of the catheter shown in Figure 1;

FIGURE 3 is an enlarged cross-sectional view taken along the line 3-3 of Figure 2;

FIGURE 4 is a schematic diagram showing representative ray paths of light radiation for a spherical lens of the type used for the invention;·

FIGURE 5 is a cross section of a lens-fiber assembly with a reinforcing collar in place;

FIGURE 6 is a perspective view of the assembly of FIGURE 5;

FIGURE 7 is a perspective view of a second alternative arrangement for the reinforcing collar;

FIGURE 8 is a perspective view of the second alternative embodiment of the reinforcing collar;

FIGURE 9 is a perspective view of a third alternative configuration of the reinforcing collar;

FIGURE 10 illustrates the arrangement for a first preferred means of joining a lens on a fiber end; and

FIGURE 11 illustrates the arrangement for a second preferred means of forming a lens on a fiber end;

## DETAILED DESCRIPTION OF THE INVENTION

In general, the intravenous catheter with laser cutting capabilities according to the present invention consists of a flexible elongate tubular member having proximal and distal extremities. The tubular member has at least one lumen extending therethrough. An optical fiber extends through the lumen and has a distal extremity which extends beyond the distal end of the tubular member. A ball-like lens tip larger than the diameter of the optical fiber is secured to the distal extremity of the optical fiber. A reinforcing collar encloses the distal end of the optical fiber, abuts the lens at the collar's distal end and is adhesively attached to the lens and the fiber. In a preferred embodiment, an inflated balloon is carried by the distal extremity of the tubular member. Means is carried by the tubular member forming a flow passage which is in communication with the interior of the balloon for inflating and deflating the balloon. In some embodiments, a variety of means to stabilize the catheter and the optical fiber such as coils, braids and tubes may be used.

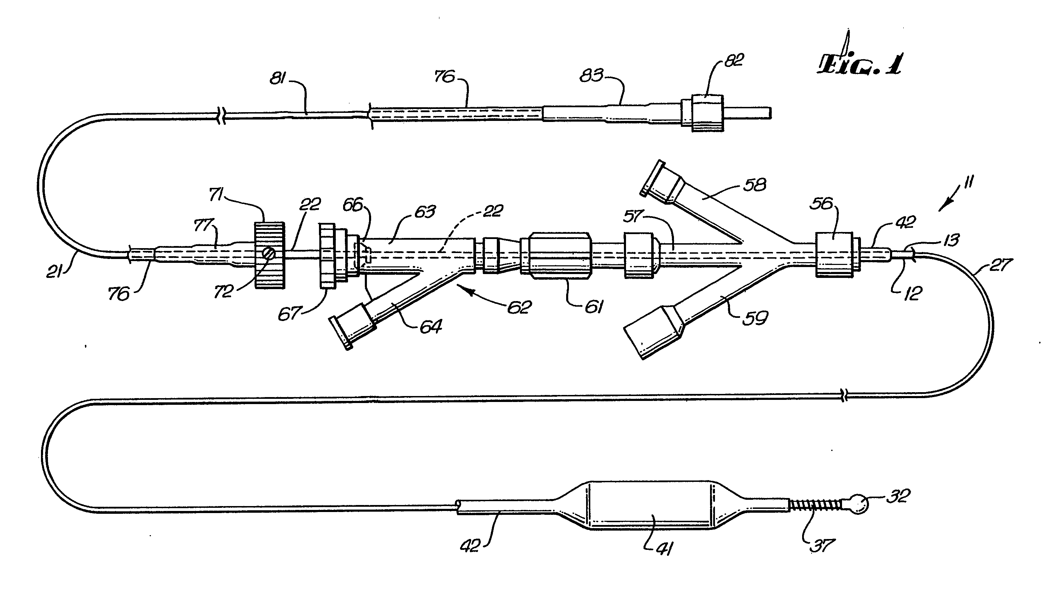

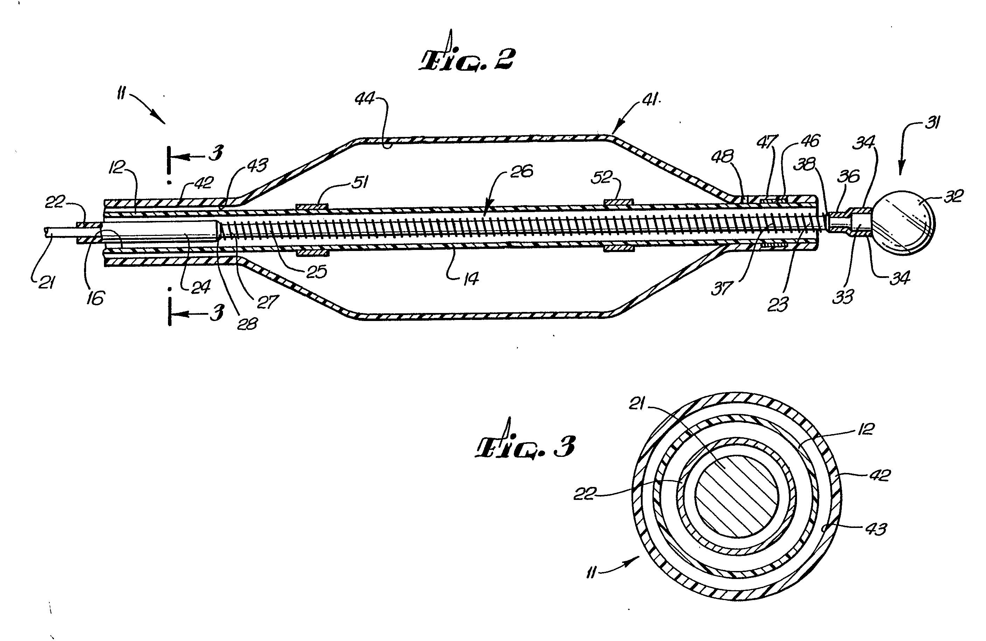

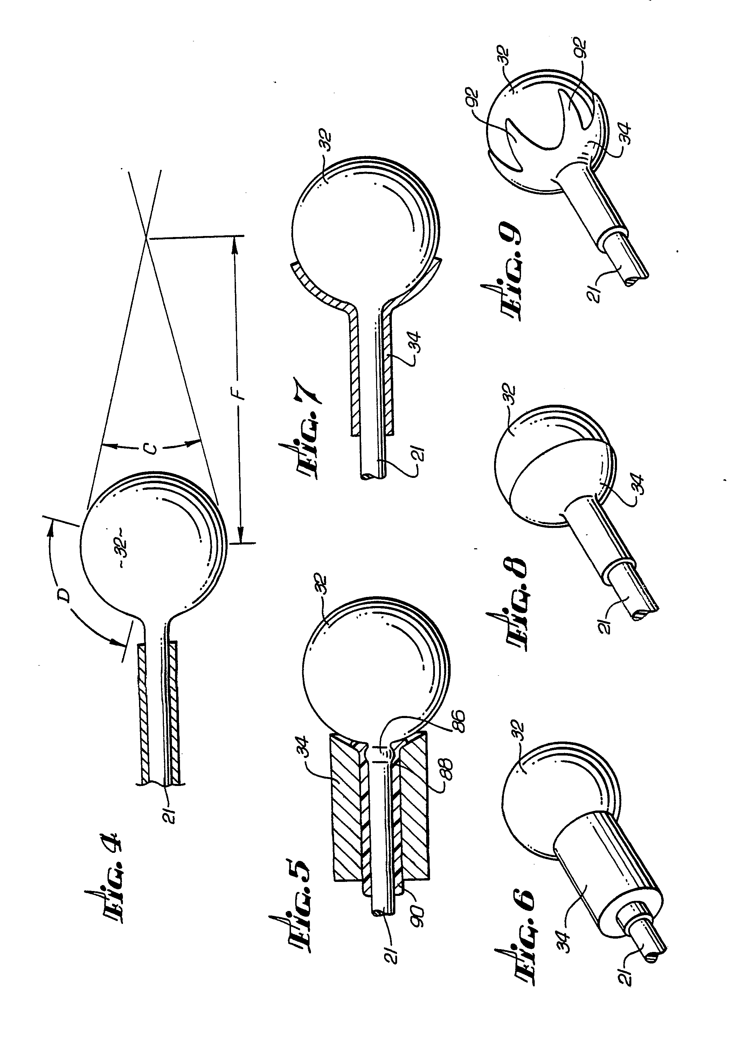

More particularly, as shown in FIGS. 1 through 3, of the drawings, the intravenous catheter 11 with laser cutting capabilities consists of an elongate flexible tubular member 12 having proximal and distal extremities 13 and 14. The tubular member 12 can be formed of a suitable material such as polyethylene tubing. The tubular member 12 can be of various sizes depending on the size of the vessel it is desired to negotiate with the catheter. By way of example, the tubular member 12 can have an outside diameter of 1.27mm (.050 inches) and an inside diameter of 1.02 mm (.040 inches). The tubular member 12 is provided with a lumen 16 extending the length thereof.

A fiber optic element or assembly 21 is provided which is adapted to extend through the lumen 16. The element can have a suitable diameter such as .3 mm (.012 inches) and can have a suitable length as, for example, a length of approximately 400 cm. In order to give additional rigidity to the fiber optic element 21 and to enhance its pushability a proximal portion inside the catheter and slightly distal of the fiber optic element is disposed within a metallic tube 22 formed of a suitable material such as stainless steel. The stainless steel tube, which may also be called a hypotube, encloses a fiber optic element 21 having an outside diameter of .30 m (.012 inches). The hypotube can have an inside diameter of .33mm (.013 inches) and an outside diameter of .76 mm (.030 inches). The hypotube 22 can have a suitable length as, for example, 80 cm. The distal extremity 23 of the fiber optic element 21 can extend a suitable distance as, for example, 10 cm beyond the distal extremity 24 of the hypotube 22. In a preferred embodiment, in order to provide sufficient flexibility for the distal extremity 23 of the fiber optic element 21 and to make it capable of withstanding bending, a coil 26 of suitable material such as stainless steel wire of a suitable diameter as,for example, .076 mm (.003 inches) is wound onto the distal extremity 23 of the fiber·optic element 21 with densely packed turns. The proximal extremity 27 of the coil 26 is secured to the distal extremity 24 of the hypotube 22 by suitable means such as an epoxy 28. The densely packed turns of the coil 24 are packed with each turn abutting another turn throughout the length of the coil 26 which extends over the distal extremity 23 of the fiber optic element 21.

A ball-like lens tip assembly 31 is provided which is secured to the distal extremity 23 of the fiber optic element. The ball-like lens tip assembly 31 consists of a ball 32 formed of a suitable material such as silica which is bonded to a silica stem 33 having a matched index of refraction. A metallic cup-like member 34 formed of a suitable material such as gold or stainless steel extends over the stem 33 and is bonded to a short metallic tube 36 formed of a suitable material such as stainless steel and having an inside diameter of .33mm (.013 inches) and an outside diameter of .46 mm (.018 inches). The tube 36 extends to the stem and is secured to the distal extremity 37 of the coil 26 and is bonded thereto by suitable means such as an epoxy 38. The stem 33 is secured to the distal extremity 23 of the fiber optic element 21. A suitable coating such as a polyimide coating can be applied to the exterior surface of the tubular sleeve 36 and the metallic cup-like collar member 34. Similarly, a suitable coating such as polyimide may be used to recoat the fiber-lens interface area after fusing of the lens to the fiber, which destroys the original polyimide coating on the fiber. The lens 32 can have a suitable diameter as, for example, from 1.2 to 3.0 mm. However, it should be appreciated that, if desired, larger lenses can be utilized. The lens, if desired, can have a diameter which is larger than the inside diameter of the tubular member 12 which forms the lumen 16 which makes the fiber optic assembly non-removable.

In a preferred embodiment, an inflatable balloon 41 is carried by the distal extremity of the elongate flexible tubular member 12. The balloon 41 as shown can be formed integrally with an elongate flexible tubular member 42 which extends coaxially of the elongate flexible tubular member 12. The balloon 41, if desired, can be formed as a separate element and bonded to the tubular member 42. An annular flow passage 43 is formed between the flexible tubular member 12 and the tubular member 42 and is in communication with the interior 44 of the balloon 41 so that the balloon 41 can be inflated and deflated by introducing a balloon inflation medium and withdrawing the same through the annular flow passage 43.

A marker 46 is provided at the distal extremity of the tubular member 12 and consists of a suitable radiopaque material such as a gold band having an inside diameter of .76 mm (.030 inches) and an outside diameter of .91 mm (.036 inches). The marker 46 is mounted on the exterior of the tubular member 12 and is retained thereon by heat shrinking the distal extremity 47 of the balloon 41 onto the distal extremity 14 of the elongate flexible tubular member 12 to provide a liquid-tight seal therebetween. Small vent holes 48 of a suitable diameter such as 1 mm can be provided from the interior 44 of the balloon and extending to ambient between the distal extremity 47 of the balloon and the distal extremity 14 of the tubular member 12 in the manner disclosed in United States Patent No. 4,638,805. These self-venting holes 48 bleed air from the interior 44 of the balloon 41 when it is being filled but prevent the escape of liquid from the balloon 41.

Additional spaced-apart markers 51 and 52 are provided for indicating the proximal and distal portions of the balloon 41 and are positioned within the balloon 41 on the tubular member 12 and also can take the form of gold bands or alternatively in the form of platinum wire wrapped onto the tubular member 12.

The proximal extremity 13 of the tubular member 12 as well as the tubular member 42 are secured to a three arm adapter 56 which is provided with a central arm 57 and side arms 58 and 59. The central arm 57 is in communication with the lumen 16 of the tubular member 12. The side arm 58 is in communication with the annular flow passage 43 in the tubular member 42. The other side arm 59 is in communication with the central lumen and can be used for perfusion.

A rotary hemostatic valve 61 is mounted on the central arm 57 and has the fiber optic element 21 with its hypotube 22 covering the same extending through the same. A two arm adapter 62 is mounted on the rotary hemostatic valve 61 and is provided with a central arm 63 and a side arm 64. The hypotube 22 with its fiber optic element 21 extends through the central arm 63 and through an O-ring 66 mounted within the central arm. They also extend through a knob 67 which is adapted to engage the O-ring to compress the same to form a liquid-tight seal between the O-ring and the hypotube 22. The side arm 64 is in communication with the central arm 63 and can be utilized for introducing a flushing medium such as a saline solution into the catheter 11.

A torque knob 71 is mounted on the hypotube 22 and is secured thereto by a set screw 72 and can be utilized for pushing the hypotube 22 and the fiber optic element 21 carried therein. The fiber optic element 21 extends beyond the torque knob 71 and is encased in an elongated flexible tubular member 76 formed of a suitable material such as polyethylene having a suitable diameter such as an inside diameter of .05 mm (.0020 inch) and an outside diameter of .1 mm (.0040 inches). Also, it can have a suitable length as, for example, a length of approximately 50 cm. A length of heat shrinkable tubing 77 can be provided on the tubular member 76 for making the transition from the torque knob 71 and to provide additional reinforcing. The proximal extremity 81 of the fiber optic element 21 is secured to a suitable connector as, for example, a conventional male SMA connector 82. A length of heat shrinkable tubing 83 is provided for establishing reinforcing at the point at which the tubular member 76 is connected to the connector 82.

Operation and use of the balloon dilatation catheter 11 with laser cutting capabilities may now be briefly described as follows. Prior to insertion of the catheter 11 into the body of the patient, the balloon 41 should be inflated with liquid to ensure that all air has been removed therefrom. This can be accomplished by inserting the balloon inflation medium through the side arm 58 and introducing it into the interior 44 of the balloon 41 to cause any air therein to be expelled through the vent holes 48. The holes 48, as explained previously, are of a size so that they permit the escape of air but prevent the escape of liquid from the interior 44 of the balloon 41. A guiding catheter (not shown) is then inserted into the vessel of the patient to be utilized for guiding the catheter 11. Thereafter if the ball is smaller than the catheter lumen, with the fiber optic assembly 19 removed from the catheter 11, a guidewire of a conventional type can be inserted into the central lumen 57 in the three arm adapter and extended through the lumen 16. A guidewire then can be utilized in a conventional manner to advance the catheter 11 beyond the guiding catheter. The guidewire can be advanced into the stenosis and then the catheter 11 can be advanced so that the balloon 41 is within the stenosis. Alternatively, the device may be used without a guidewire, the lens tip and relatively rigid portion of the catheter combining to provide the necessary mechanical characteristics used to guide the catheter to an area of stenosis. A suitable balloon inflation medium can then be introduced into the side arm 58 to inflate the balloon 41. As another operational alternative, a guidewire may be inserted into the same lumen that houses the optical fiber. The guidewire is then used to guide the end of the catheter to the artery containing the lesion. After the catheter is in place, the guidewire is retracted prior to lasing of the lesion. For this embodiment the two arm hemostatic valve is replaced by a three arm hemostatic valve.

After the balloon 41 has been positioned in the stenosis, the balloon 41 can be inflated by introducing a balloon inflation medium through the side arm 58 to inflate the balloon for a suitable time at the desired pressure. After the balloon inflation has been utilized to create a larger opening in the stenosis, the balloon 41 can be deflated and withdrawn.

If it is desired to perform additional operations on the stenosis by laser cutting, the guidewire can be removed and the fiber optic assembly 19 incorporating a lens end smaller than the lumen 16 can be inserted through the lumen 16 until the lens 32 extends beyond the distal extremity of the catheter 11. The lens 32 is self guiding and greatly reduces possible perforation of the side wall of the vessel being negotiated. The lens 32 facilitates negotiation of tortuous vessel after the lens 32 has been appropriately positioned.

If the lens 32 is larger than the catheter lumen 16, as is the case in one preferred embodiment, a separate guidewire cannot be used. The fiber optic assembly can then be assembled into the catheter 11, as shown in FIG. 2, so that is is a part of the catheter 11. The fiber optic assembly 13 with its lens 32 can act as a guidewire for guiding the catheter 12 through tortuous vessels.

Laser energy can then be introduced by a laser (not shown) connected to the SMA connector 82 causing an appropriate amount of energy to be directed down the fiber optic assembly 19 and into the lens 32 to cause ablation of the atheromatous tissue in the stenosis while at the same time minimizing adjacent thermal injury to the vessel. During this procedure, the tissue being irradiated can be immersed in a suitable saline solution by introducing the saline solution into the side arm 64 of the two arm adapter 62. Alternatively, the laser irradiation may occur in the presence of blood in the region adjacent the lens, since one advantage of the present invention is that the radiation pattern of the lens, which is concentrated in the immediate distal area of the lens concentric with the lens, does not have to be actively aimed and may be placed in close contact with the stenosis to be removed. Thus, there is very little blood between the lens and the stenosis to be irradiated by laser energy and absorb the energy emitted.

The lens of the present invention may advantageously be of spherical form and is sized to be substantially larger than the diameter of the optical fiber to which it is connected. In practice, it has been found that lens diameters of approximately one to three millimeters are effective in focusing the laser light on the area of stenosis located in front of the lens and may be used to advantage in assisting the advance of the catheter through an area of stenosis which has been enlarged due to laser cutting. Furthermore, the large lens in comparison to the diameter of the fiber provides an optical characteristic which maximizes the forward projection of light energy and reduces the radial emission of light with a subsequent reduction in the probability of damaging the arterial wall.

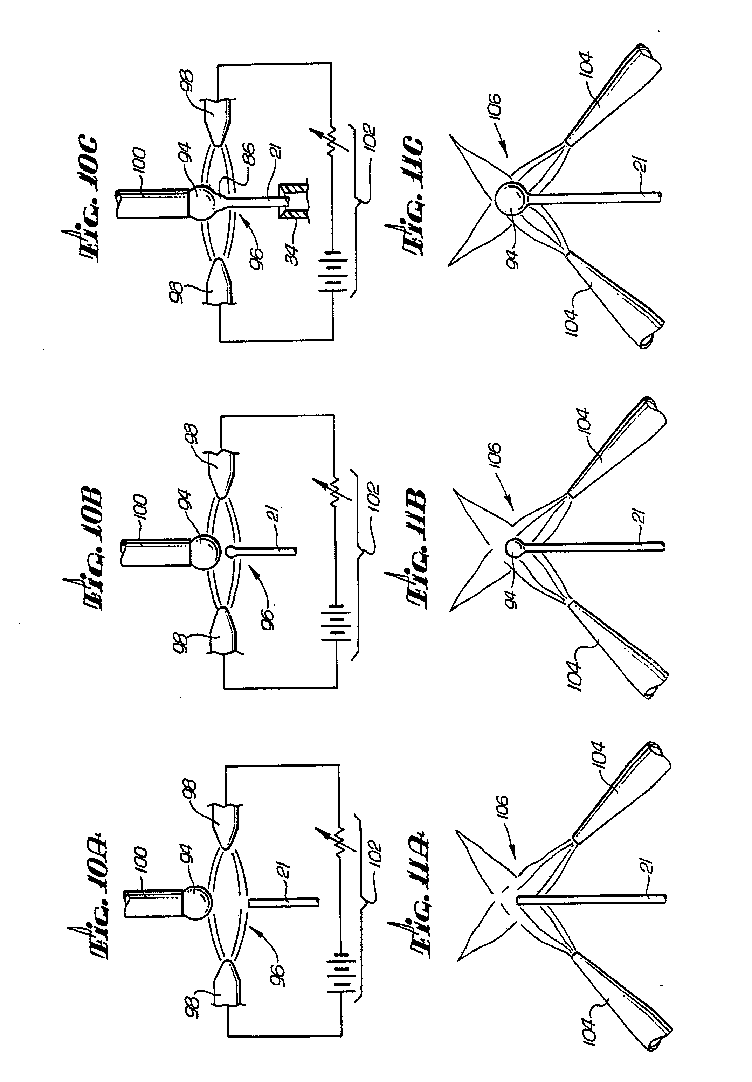

FIG. 4 illustrates a cross-sectional view of an essentially spherical lens of the type used in the present invention attached to an optical fiber through which light from the laser is conducted. As shown, the light from the lens is focused in a cone C to a distance F in front of the lens. In practice it has been found that nearly 100% of the light from such a lens is focused within such a cone, provided that the lens is at least 2 to 3 times the diameter of the optical fiber. In the remainder D of the angular area surrounding the lens, much less than 10% of the optical energy is dissipated.

The present invention also incorporates a reinforcing collar which may be configured in a variety of ways to reinforce the juncture between the relatively large lens and the optical fiber. FIG. 5 is a cross-sectional view of a lens and fiber according to the invention, illustrating the reinforcing collar in place around the fiber and lens and providing support to the interface between them. As illustrated, the lens 32 may be fused or formed on optical fiber 21 forming an interface 86. In the event that the lens 32 is first formed independently and then fused to the fiber 21, a procedure that is advantageous if a preformed, non-spherical lens is used, a displaced area 88 will be found near the interface. This displaced area 88 serves as a transition between the relatively large lens and the fiber and decreases the probability that a fracture will occur at the interface. A metal collar 34 is placed around the fiber 21 and abuts the lens 32. In an alternative embodiment, illustrated in FIG. 5, the collar is a hollow, essentially cylindrical member that is beveled at the distal end to mate with the proximal end of the lens. Epoxy material 90 is placed in the area between the collar and the lens and fiber to adhesively bond them together.

In practice, it has been found that it is advantageous to fabricate the collar 34 of 304 Stainless Steel and to utilize TRICON FDA-2 epoxy as an adhesive. Prior to adhering the collar to the fiber and lens, the transition area 86 may be recoated with a polyimide coating similar to that with which the original fiber was clad. After the collar is bonded to the lens and fiber to form an assembly, the assembly may be coated with polydimethyl siloane and heat cured at 300° C to form Cyctic Siloanes. This coating reduces adhesion of proteins formed during the lasing process and increases the apparent aperture of the lens during a procedure. In one alternative embodiment, a coating of gold or aluminum may be deposited on the proximal end of the lens in the transition area to improve the thermal characteristics, especially heat transfer, between the lens and the fiber.

FIG. 6 is a perspective view of the embodiment of FIG. 5 illustrating the relationship of collar 34 to lens 32. Those skilled in the art will recognize that while a particular geometry of lens, fiber and transition are presented, other geometries and relationships may prove advantageous for various laser angioplasty procedures. Further, while an essentially spherical lens is illustrated, other lens configurations that are larger than the fiber may be used and the lens shape may be configured to suit specific requirements of individual procedures in laser angioplasty or other _in vivo_ laser ablation techniques.

FIG. 7 is a cross-sectional view of an alternative preferred embodiment in which the reinforcing collar 34 is relatively thin and is joined to cradle the proximal end of the lens 32. This configuration has less thermal mass and weight than the configuration of FIGS. 5 and 6. In some embodiments of the invention, this configuration can provide a means of controlling the thermal gradients across the interface of the lens and the fiber, thereby reducing the chance that thermal anomalies may contribute to the fracture of the interface between the lens and the fiber.

FIG. 8 is a perspective view of an alternative configuration of the reinforcing collar of FIG. 7 showing its extension around a portion of the lens. The amount by which the collar 34 extends around the lens 32 may be varied, depending upon the geometric relationship between the diameters of the fiber and the lens and the amount of contribution that the collar is to make to control of reflected energy and heat transfer across the interface.

FIG. 9 is a perspective of another alternative configuration of the collar 34 illustrating how the collar 34 can be somewhat lower in mass than the embodiment of FIG. 8 and have fingers 92 which extend around a portion of the lens 32 to increase the retention of the lens in the collar without unnecessarily increasing the thermal mass.

In each case described above, since the radiation is primarily concentrated in a small angle in front of the distal end of the lens, relatively small amounts of energy impinge upon the collar and thus there is a relatively small amount of heating of the collar by the laser energy transmitted through the fiber, thereby reducing the chance that there will be a degradation of the adhesive between the collar and the lens. Thus, the invention provides, by means of the collar, the configuration of the lens, the coatings that may be used, and the relative dimensions of the lens and fiber, the means to control thermal gradients and strength of the interface between the lens and the fiber, thereby reducing the risk of fracture of the interface.

FIG. 10 illustrates one means of fusing a spherical lens 32 of the type employed in certain embodiments of the invention to the end of an optical fiber 21. In practice, as illustrated in FIG. 8a, silica sphere 94 is positioned adjacent optical fiber 12 in the proximity of a work area 96 between electrodes 98 of an arc emitting apparatus by being held in a vacuum probe 100. A variable voltage supply 102 is used to propagate a spark between electrodes 98 into work area 96. As illustrated in FIG. 8b, the silica sphere and the optical fiber are brought into close proximity with one another within the arc and localized melding of the sphere and the fiber result in fusion of the fiber and the sphere with an intermediate area 86 representing an area of cross-section intermediate between the diameter of the sphere and the fiber. After the assembly is cooled, the collar 34 may be slipped over fiber 12 and adhesively bonded as described above.

An alternate means of forming the lens is illustrated in FIG. 11 which shows the optical fiber 21 placed between two gas flames fed by an electrolytic gas generator of the type commercially. designated MG-300. Using this procedure, the gas generator is used to feed hydrogen gas to two nozzles 104 which sustain flames in the work area 106. The gas generator generates hydrogen gas that may be used to heat the distal end of fiber 21 to form the lens. However, if pure hydrogen were used, the flame from nozzles 104 would be too hot to properly form the lens. Therefore, an additive such as a mixture of acetone and alcohol may be used to lower the flame temperature and assist in a more consistent formation of lenses. While a variety of moderators may be useful for the control of flame temperature, it has been found that a mixture of acetone and methyl alcohol is economical and produces the desired reduction in flame temperature. The fiber is clamped in a fiber holder (not shown) and the distal tip of the fiber is set in line with the flames from the nozzles. The nozzles are inclined upward to keep the area of highest temperature away from the proximal end of the fiber, thus keeping the lens liquid during the entire process until it is removed from the flame. The tip of the fiber is heated until it melts and while heating, the fiber is slowly moved upward until a lens tip 34 is allowed to grow to approximately any desired diameter. Thereafter, the gas generator is turned off and the lens tip is measured with a micrometer. The above procedure is repeated until a spherical lens tip of the appropriate diameter is produced. Using this method, there is a smooth but rapid transition from the diameter of the optical fiber to the lens and it is important to use a collar of the type described above to reinforce this junction, enhance pushability, and prevent a fracture of the interface between the optical fiber and lens. The above procedures have been found to produce connections of high optical quality which when combined with the transparency of the lens results in low light loss during the laser cutting process.

From the above, it should be appreciated that various types of procedures can be accomplished with the laser cutting catheter of the present invention in performing in vivo laser angioplasty. If a vessel is totally occluded, the lens tip can be utilized for ablating the tissue forming the stenosis to form at least a small opening therethrough. Thereafter, if desired, the balloon 41 can be advanced into the opening formed in the stenosis and further enlarged by inflating the balloon 41 for a desired time at an appropriate pressure. The lens tip 32, in addition to making it possible to deliver substantial amounts of energy to the area in which ablation is to occur, is also effective in angioplasty procedures in that it minimizes adjacent thermal injury. In addition, the lens tip 32, because of its rounded configuration, inhibits perforation of the vessel upon which the procedure is being performed.

It is apparent from the foregoing that there has been provided a laser cutting catheter which has capabilities which can be utilized in in vivo angioplasty. Additionally, the laser cutting catheter can be provided with balloon dilatation means that may be used to enlarge the channel cut in the stenosis by laser radiation. The lens tip is self guiding, which facilitates its movement in tortuous vessels while limiting the possibility of mechanical perforation of the vessel. Furthermore, the lens tip self guiding feature, in combination with rigidity enhancing features described above, may in certain instances eliminate the necessity to provide a separate guidewire for the catheter.

Because of the configuration of the lens and its resultant axial propagation pattern, the lens may be brought into close proximity or contact with the stenosis for laser ablation, thereby providing the capability to perform laser cutting while a normal flow of blood is present and with reduced probability that the wall will be perforated. The coating of the distal end of the lens-fiber assembly with siloxanes reduces the adherence of proteins produced in the laser process, thus providing a larger effective aperture for the lens.

While particular forms of invention have been illustrated and described, it will be apparent that various modifications can be made without departing from the spirit and scope of the invention. Accordingly, it is not intended that the invention be limited, except as by the appended claims.

## Claims

1. An intravascular catheter with laser cutting capabilities which comprises:
a flexible elongate tubular member having proximal and distal extremities, said tubular member having at least one lumen extending therethrough;
an optical fiber assembly having a distal and a proximal end, said optical fiber assembly extending through said lumen, said distal end of said optical fiber assembly having a lens attached thereto, said lens having a diameter substantially larger than the diameter of the light conductive portion of said optical fiber assembly; and
reinforcing means attached to said lens and said optical fiber assembly to thereby increase the strength of the juncture therebetween.

2. The intravascular catheter with laser cutting capabilities of claim 1 which further comprises:
an inflatable balloon carried by said tubular member near the distal end thereof; and
means carried by said tubular member to provide a passage for fluid for inflating and deflating said balloon.

3. The intravascular catheter with laser cutting capabilities of any one of the preceding claims wherein said lens further comprises an essentially spherical lens.

4. The intravascular catheter with laser cutting capabilities of any one of the preceding claims wherein said reinforcing means further comprises a collar which fits around the distal portion, said light conducting portions of said optical fiber and mates with the proximal portion of said lens.

5. The intravascular catheter with laser cutting capabilities of any one of the preceding claims wherein said lens is fused to said optical fiber.

6. The intravascular catheter with laser cutting capabilities of any one of the preceding claims further including a reinforcing tube which encloses a substantial portion of said fiber optic element to increase rigidity and enhance pushability.

7. The intravascular catheter with laser cutting capabilities of claim 6 wherein said reinforcing tube is made of metal.

8. The intravascular catheter with laser cutting capabilities of any one of the preceding claims wherein said lens and the core of said optical fiber assembly and said lens have substantially similar refractive indexes.

9. The intravascular catheter with laser cutting capabilities of claim 8 wherein said lens is formed by controlled melting of the distal end of said optical fiber assembly.

10. The intravascular catheter with laser cutting capabilities of any one of the preceding claims wherein said lens is formed integrally with said light conducting portion of said optical fiber.

11. A method of fabricating a fiber optic element with an essentially spherical lens attached to the distal end thereof which comprises:
clamping an essentially spherical bead in a first clamping means in a work area between electrodes capable of sustaining an electrical plasma when suitably excited;
clamping an optical fiber in a second clamping means in said work area, with a free end of said fiber protruding into the work area;
generating a plasma between said electrodes;
moving said free end of said fiber and said bead into close proximity to one another in said plasma to thereby cause the free end of said fiber and a portion of said bead to become molten; and
fusing the molten end of said fiber to a molten portion of said bead.

12. The method of claim 11 which further comprises the step of:
heating the end of said fiber prior to joining said fiber and said bead to thereby create an area of intermediate diameter between said bead and said fiber.

13. A method of fabricating an essentially spherical lens end on a fiber optic element, said method comprising:
clamping an optical fiber in a clamp with the free end extending into a work area which includes a plurality of nozzles fed by a gas which burns with a low residual flame;
causing the nozzles to propagate flames into said work area;
moving the free ends of said fiber into the area heated by said flames;

holding said free end of said fiber in said flames until a spherical tip is formed on said free end of said fiber by melting of said fiber and the application of surface tension; and
removing said fiber from said flames to cool.

14. The method of claim 13 which further comprises the step of repeating the above procedure until a sphere of the appropriate size is grown.

15. The method of claims 13 or 14 wherein said low residual gas is hydrogen.

16. The method of claim 15 which further comprises adding a moderating chemical to said hydrogen to thereby produce a flame having a lower temperature than that produced by hydrogen alone.

17. The method of claim 16 wherein said moderating chemical is a mixture of acetone and alcohol.

18. The method of claim 15 wherein said means to propagate a flame into said work area further comprises hydrogen generating means.

19. A method of attaching a preformed lens to the end of an optical fiber which comprises:
clamping an optical fiber in a first holding device so that a free end of said fiber extends into a desired work area;
placing a pre-formed lens into a second holding device in said work area;
causing an electric arc to be drawn between said lens and said fiber;
causing said lens and said free end of fiber to be adjacent to one another in close proximity to said arc; and
positioning said lens and said fiber in said arc for a period of time sufficient to fuse said lens and said fiber at their interface.

_Fig. 1_

EP 0 355 200 A1

Fig. 2

Fig. 3

EP 0 355 200 A1

Fig. 4

Fig. 5

Fig. 7

Fig. 6

Fig. 8

Fig. 9

EP 0 355 200 A1

Fig. 10A

Fig. 10B

Fig. 10C

Fig. 11A

Fig. 11B

Fig. 11C

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 273 109 (ENDERBY) <br> * Column 3, lines 11-43; column 4, lines 25-27; column 5, lines 1-6; figure 2 * <br> --- | 1-3,5,6 ,8-10 | A 61 B 17/32 |
| Y | EP-A-0 217 165 (FOX) <br> * Page 17, lines 15-24; figure 4 * <br> --- | 1-3,5,6 ,8-10 | |
| Y | EP-A-0 142 026 (CONSIGLIO NAZIONALE DELLE RICERCHE) <br> * page 9, lines 1-16; figures 2,3 * <br> --- | 3,5,8- 10 | |
| Y | EP-A-0 025 728 (THOMSON) <br> * Page 4, lines 4-25; figures 2,3C * <br> --- | 11,12, 19 | |
| Y | FR-A-2 548 391 (COMPAGINIE GENERALE D'ELECTRICITE) <br> * Page 6, lines 2-22; figure 8 * <br> --- | 11-15 | |
| Y | EP-A-0 232 918 (AMERICAN TELEPHONE & TELEGRAPH CO.) <br> * Column 3, lines 41-48 * <br> --- | 13-15 | TECHNICAL FIELDS SEARCHED (Int. Cl.4) |
| Y | US-A-4 743 283 (BORSUK) <br> * Column 2, lines 15-27; figure 1 * <br> ----- | 19 | A 61 B <br> B 23 K <br> A 61 F <br> C 03 B |

The present search report has been drawn up for all claims

| Place of search | Date of completion of the search | Examiner |
|---|---|---|
| THE HAGUE | 13-11-1989 | MOERS R.J. |